# EUROPEAN PATENT APPLICATION

(11) **EP 2 497 761 A1**
(43) Date of publication of application: **12.09.2012**
(21) Application number: 12155009.9
(22) Date of filing: 10.02.2012
(51) Int. Cl.: C07C 51/64

(54) **Process for the purification of anthraquinone derivatives**

(30) Priority: 11.02.2011 IT TO20110120
(71) Applicant: ICROM S.p.A., 20122 Milano, MI (IT)
(72) Inventor: Leone, Mario, 26831 Casalmaiocco (LO) (IT); Daverio, Paola, 20852 Villasanta (MB) (IT); Cipriano, Marco, 20832 Desio (MB) (IT)
(74) Representative: Witek, Rafal

(57) **Abstract**

The present invention relates to a process for obtaining anthrakinone derivatives used in therapy with a toxic impurity content <30ppm; preferably < 5ppm; even more preferably <2ppm.

## Description

### FIELD OF THE INVENTION

The present invention relates to a process for obtaining therapeutically-active anthrakinone derivatives with a content of toxic impurities < 30 ppm; preferably < 5 ppm; more preferably < 2 ppm.

### BACKGROUND OF THE INVENTION

Therapeutically-active anthrakinone derivatives are known. Rein (Ia) and other 1,8-diacyl anthraquinone derivatives, among which diacerein (Ib), disclosed in GB 1578452, are known thanks to their therapeutic properties in the treatment of degenerative diseases of the joints and/or of the connective tissue, such as osteoporosis, rheumatoid arthritis, and in the long term treatment of osteoarthritis (Malterud et al., Antioxidant and Radical Scavenging Effects of Anthraquinones and Anthrones, Pharmacol., (Basel), 47 Suppl. 1; 77-85, 1993).

Rein is also useful in other pharmacological activities such as the bacteriostatic activity (Wang et al., Biochemical Study of Chinese Rhubarb - Inhibition of Anthraquinone Derivatives on Anaerobic Bacteria; Zhongguo Yaoke Daxue Xuebao, 21(6), 354-57, 1990), and the anti-cancer activity, for example in combination with Adriamycin (Fanciulli et al., Inhibition of Membrane Redox Activity by rein and Adriamycin in Human Glioma Cells, Anti-carcinogenic drugs, 3(6), 615-21, 1992).

Rein (Ia) is a compound of formula (I) 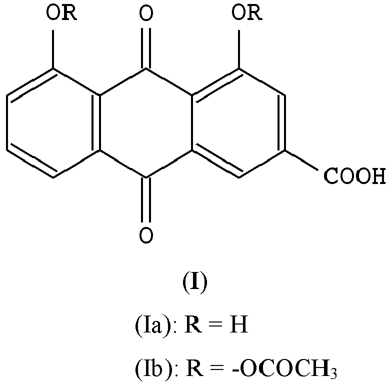 wherein R is H.

Diacerein (Ib) is a compound of formula (I), wherein R is -OCOCH₃ chemically known as 1,8-diacetoxy-3-carboxy anthraquinone, commercially available under various trade names, for example Artrodar^{®}.

Synthesis processes for obtaining anthraquinone derivatives are known.

French Pat. No. 2508798-B1 and Belgian Pat. No. 875945 disclose the preparation of diacerein via dissolution of 1,8-dihydroxyanthraquinone-3-carboxylic acid in excess acetic anhydride, in the presence of sulfuric acid as a catalyst.

1,8-Dihydroxy-anthraquinone-3-carboxylic acid occurs either free or as a glucoside in several plants, e.g. in Senna leaves. It is also prepared from chrysophanic acid diacetate (The Merck Index, 11th Ed., 1989, Merck & Co., Inc., Rahaway, N.J., USA, 8175 and 2263) and by oxidation of the corresponding 3-hydroxymethyl derivative, i.e. 1,8-dihydroxy-3-hydroxymethylanthraquinone (aloe-emodin), with chromic anhydride ("Sostanze formaceutiche", a translation into Italian and revision by R. Longo, OEMF, Milano, 1988, p. 596 of "Pharmazeutische Wirkstoffe, Synthesen, Patente, Anwendungen", A. Kleemann, J. Engel, George Thieme Verlag, Stuttgart-New York, 1982-1987).

The known processes include the use of a very toxic substance such as chromic anhydride. As to the chromium derivatives, see, for example, what is declared by the European Community according to which the chromic anhydride "can cause cancer by inhalation" (see the EEC Directive 94/95/CE of July 30, 1996,).

Furthermore, Rein and diacerein obtained by the prior art processes contain - as a byproduct - considerable amounts of aloe-emodin, which has mutagenic activity even in amounts of 70 ppm.

According to the current good manufacturing practices (GPM), pharmaceutical marketed products must contain the lowest possible amount of impurities (this is based on the assumption that substances devoid of a pharmaceutical effect are toxic, for the mere fact that they are chemicals).

It is therefore important to find methods for the purification of the anthraquinone derivatives which allow to obtain contents of impurities within the allowed limits.

The process for the purification of rein or its derivatives aims at obtaining a product substantially free from impurities and, in particular, at eliminating aloe-emodin which is supposed to be mutagenic. It also aims at obtaining a product with a chromium content lower than 50 ppm.

Some Regulatory Agencies, such as the French Agency for pharmaceutical Products (AFSSAPS), have determined that rein or its derivatives must contain a maximum of 2ppm aloe-emodin and partially acetylated derivatives of aloe-emodin.

The ICH guideline (International Conference on Harmonisation of Technical Requirements for Registration of Pharmaceuticals for Human Use) "Guideline on the limits of genotoxic impurities" provides that, in the absence of sufficient experimental evidence for the determination of a threshold of toxicity, the Toxicological Threshold Concern (TTC, "Soglia di allarme tossicologico"), must be applied to the potentially genotoxic impurities, which the guideline itself has determined to be of 1,5 µg/person/day. Therefore, for a therapeutic dose of 50 mg/day, a 30 ppm residual level of aloe-emodin is to be considered safe for human consumption.

A certain number of processes for the purification of rein or crude diacerein is known.

For example, EP 520414 B1 discloses the synthesis of diacerein by oxidation of the rein-9-anthrone-8-glucosyde compound, hydrolysis of the glucosyde and acetylation. The synthesis step is followed by the purification of aloe-emodin through a liquid-liquid separation between an organic and an aqueous phase at pH 6.5-7.5. Diacerein is recovered by acidification and is optionally purified by crystallization from ethyl lactate.

EP 636602 B1 discloses a process for diacerein preparation by acetylation of aloin, oxidizing of the acetylated product and purification of the crude diacerein by crystallisation from toxic solvents: 2-metoxiethanol or N,N-dimethylacetamide.

EP 0928781 B1 discloses a process for the preparation of rein and diacerein, by oxidation of aloe-emodin and its triacil derivatives, with nitrous acid salts, dangerous and highly toxic reagent, followed by acylation of the rein thus obtained.

EP 96900687 B1 discloses a process wherein diacerein is suspended in a mixture of organic solvents and water, a solution is obtained by addition of a tertiary amine, impurities are filtered off, diacerein is again precipitated as a salt with an alkali/alkaline-earth metal, and then is dissolved in slightly acid water.

It has, however, been observed that the solubilization of the diacerein salt in water, prior to acidification with weak acids, causes partial de-acetylation with consequent formation of one/two impurities (rein and mono-acetylated derivatives) causing a loss of the product titre of about 0.5-5%. It is known that protection of the hydroxyl groups by acetylation is reversible, as hydrolysis occurs even in only slightly acidic or basic media.

Diacerein, in particular, remains stable when changing from a neutral to an acid medium, but it undergoes a fast de-acetylation in even slightly basic medium: in particular, the basicity produced by the diacerein sodium salt is sufficient to induce hydrolysis. The subsequent re-acetylation step carried out with acetic acid, acetic anhydride or acetyl-chloride is complex, incomplete and reduces the final yields.

WO 9856750 discloses a process for the preparation of diacerein characterized by the purification of crude diacerein via salificaton with the organic amine triethylamine, precipitation of the organic alkali salt by addition of the sodium 2-ethylesanoate alkali salt and subsequent conversion to the acid form with diluted acids.

However no details are provided on the obtained purity degree of diacerein.

EP 1177164 B1 discloses a process for purifying diacerein by crystallization of the crude product with acetic acid and acetic anhydride, or with only acetic anhydride, followed by treatment of the solution with ethylenediaminotetraacetic acid (EDTA) to remove any chromium traces. Diacerein is obtained in 80/90% yields having 99,7% minimum purity, an aloe-emodin content < 50ppm and a chromium content < 15 ppm.

EP 1567474 B1 discloses a process for obtaining diacerein with an aloe-emodin content < 100ppm. The process comprises subjecting an aqueous-organic solution of a diacerein salt with a weak base to discontinuous or continuous extraction with a water immiscible or sparingly water-miscible solvent, and precipitating of the pure diacerein by acidification.

WO 2009106909 discloses a process for the purification of crude diacerein wherein diacerein is repeatedly cristallized from the 1-methyl-2-pyrrolidone toxic solvent or from a mixture of 1-methyl-2pyrrolidone and a co-solvent.

Therefore, there is a need for a process aimed at obtaining diacerein with high yields and above all free from the aforesaid impurities such as heavy metals and aloe-emodin; such process comprising the use of non-toxic solvents suitable for the pharmaceutical use of the product.

An object of the present invention is to provide a simple and economic process, with few steps, for obtaining pure anthraquinone derivatives that may be used in therapy, with a content of toxic impurities, heavy metals, aloe-emodin <30 ppm; preferably < 5ppm, even more preferably < 2ppm.

### SUMMARY OF THE INVENTION

Surprisingly, the Applicant has found a new process for purifying anthrakinone derivatives of formula (I), or salts thereof

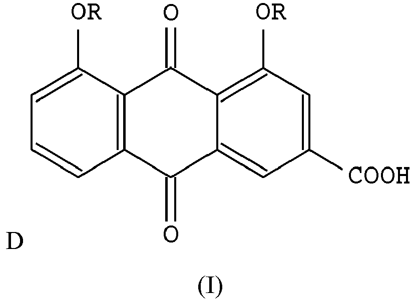

Wherein R is hydrogen, acyl.

Such process allows to obtain anthrakinone derivatives of formula (I) with an aloe-emodin content < 30ppm; a chromium content <30ppm and comprises:
a) suspending the anthrakinone derivative of formula (I) in a sulphoxide or in a mixture of a sulphoxide and another solvent;
b) adding an alkali or alkaline earth base to give the corresponding carboxylic salt;
c) separating the carboxylic salt;
d) adding a mineral acid in an organic solvent or in a mixture of an organic solvent and water to give the compound of formula (I);
e) crystallisating, in a mixture of a sulphoxide and another solvent, the compound of formula (I) as obtained in step d).

The anthrakinone derivatives of formula (I) obtained according to the process of the present invention have an aloe-emodin content preferably <5 ppm, more preferably <2ppm; a chromium content < 5 ppm, even more preferably < 2ppm.

In a further object, this invention relates to the use of a mixture of sulphoxide and another solvent as a crystallization solvent for purifying anthrakinone derivatives of formula (I).

The process according to the present invention is simple, economic and can be advantageously used on industrial scale to obtain highly purified anthrakinone derivatives of formula (I). In particular, the process according to the present invention is suitable to obtain diacerein (Ib) with the purity required by the most restrictive regulatory standards.

The diacerein obtained according to the process of the present invention can be formulated in pharmaceutical compositions and used in therapy.

### DETAILED DESCRIPTION OF THE INVENTION

All terms used in this application, unless otherwise stated, must be understood in their common meaning as known in the art. Other more specific definitions for certain terms, as used in this application, are highlighted below and are applied consistently throughout the specification and claims, unless a different definition explicitly provides a broader definition.

The term "acyl" refers to an -OCOR group wherein R is an aliphatic or aromatic group, linear or branched, containing from 1 to 12 carbon atoms, optionally substituted with one or more halogens.

Preferably R is an alkyl with 1-3 carbon atoms. Examples of acyl groups include, without limitation, acetyl, trifluoroacetyl, trichloroacetyl and the like, preferably acetyl. The term "acylating agent" refers to compounds useful for the conversion of an OH group to an -OCOR group. The acylating agent refers to an activated form of a carboxylic acid, for example, ester, anhydride, acyl halides, such as acyl chlorides.

The term "salt" refers to pharmaceutically acceptable salts, relatively non-toxic salts, suitable for use in contact with the tissues of humans and pet animals, devoid of toxicity, irritation, allergic response and the like, which possess a good benefit/risk ratio and are effective. Pharmaceutically acceptable salts are known.

For example, S. M. Berge, et al., describe pharmaceutically acceptable salts in detail in J. Pharmaceutical Sciences, 1977, 66:1-19. The salts can be prepared in situ during isolation and final purification of the compounds of the invention, or separately, by reacting the free acid with an appropriate base.

The pharmaceutically acceptable salts of the compounds according to the invention include salts with inorganic bases, for example alkali metals, such as sodium, lithium, potassium, or alkaline earth metals, for example, calcium, magnesium, and the like; or with organic bases, for example ammonia, ammonium, quaternary ammonium and amine cations, including, but not limited to, ammonium, tetramethylammonium, tetraethylammonium, alkylamines, for example methylamine, ethylamine, dimethylamine, di-ethylamine, tri-ethylamine, piperidine, piperazine, morpholyne and the like; or with amino acids Basic or with osamine, (such as meglumine), or with amino alcohol (such as 3-aminobutanole and 2-aminoethanole) and the like.

The term "aloe-emodin" refers to both aloe-emodin as such and to aloe-emodin in admixture with the corresponding mono, di, and/or tri-acetyl derivatives, by-products which may be present in the crude anthrakinones derivatives of formula (I).

The term "sulfoxide" refers to an aprotic polar solvent with a sulfoxide group R-SO-R, wherein R independently represents an alkyl group containing from 1 to 6 carbon atoms or an aryl group containing from 6 to 15 carbon atoms. Examples of sulfoxides include, without limitation, dimethyl sulfoxide, diethyl sulfoxide, dibuthyl sulfoxide, tetrahydrotiofen sulfoxide and the like, preferably dimethyl-sulfoxide.

The term "about" refers to the range of the experimental error that may occur in a measurement.

The anthrakinone derivatives of formula (I), as defined above, are obtained from commercially available aloin, by the acylation and oxidation processes known to the skilled in the art.

Aloin or barbaloin (10-glucopyranosyl-1,8-dihydroxy-3-hydroxymethyl-anthracen-0-one) is a natural substance isolated from various species of aloe, consisting of two isomers named A and B (Epimers α e β at the glycosidic residue); for the purpose of the present invention the term refers indifferently to each of the two or to mixtures of both in any ratio they are present.

Aloin is acylated by an acylating agent well known to the skilled in the art, preferably acetic anhydride, present in remarkable stoichiometric excess, preferably 30-60 molar equivalent, without the aid of any catalyst, at temperatures ranging from 30° C to 150° C, preferably at reflux temperature, even more preferably at a temperature comprised between 110° C and 140° C, for the time required to complete the reaction.

In the acylation reaction, various organic solvents can be used as diluents, for example methylene chloride, or compatible with the reaction conditions, glacial acetic acid, acetic anhydride or any other organic solvent known to the skilled in the art which is not able to react chemically with acetic anhydride itself.

The epta-acylated product can be oxidized directly in a solution without being isolated or it can be oxidized following isolation. Preferably, the acylated product is oxidized in a solution directly, without isolating the acylated intermediate. Any oxidizing agent, capable of oxidizing a primary alcohol in the corresponding carboxylic acid known to the skilled in the art, can be used.

To this purpose, we refer to a comprehensive text of organic chemistry, such as "Advanced Organic Chemistry - Reactions, Mechanisms and Structures", Jerry March, John Wiley & Sons, 3rd Ed., 1985 (in particular, par. 0-22 and 0-23, p. 346-347; par. 9-22, p. 1084).

The preferred oxidizing agent of the present process is chromic acid in molar ratios ranging from 3 to 10 moles per mole of aloin and preferably from 4 to 8 moles per mole of aloin. The whole synthesis can be carried out in a closed cycle to ensure the protection of the operators and the external environment.

The acid or basic hydrolysis of the corresponding acyl derivatives of formula (I), thus obtained, allows to obtain the anthrakinone derivatives of formula (I) wherein R is hydrogen.

The purification of the anthrakinone derivatives of formula (I), according to the process of the present invention, is characterized by the crystallization in a mixture of a sulfoxide and another solvent of the compound of formula (I), previously salified/desalted.

Surprisingly it was found that the salification step of the anthrakinone derivatives of formula (I) with an alkali or an alkaline-earth base, followed by desalting and subsequent crystallization in a mixture of a sulfoxide and another solvent, allows to obtain anthrakinone derivatives of formula (I) with a content of aloe-emodin and chromium in line with the current regulatory requirements.

It has been found that, solvents of low toxicity belonging to the sulfoxides class, for example dimethylsulfoxide, diethylsulfoxide, dibuthylsulfoxide, tetrahydrothiophene sulfoxide, and the like, preferably dimethylsulfoxide in admixture with other organic solvents of low toxicity such as ketones, for example acetone, methyl isobutyl ketone, methyl ethyl ketone, preferably acetone; alcohols, for example methanol, ethanol, isopropanol; acetates, for example ethyl acetate, butyl acetate; ethers, for example tetra hydro furan, tetra hydro pyran, and the like. They are very effective in removing residues of inorganic contaminants, such as chromium, and aloe-emodin residues from anthrakinone derivatives of formula (I), as above described, whether they are in the acid form or in the form of alkali or alkaline-earth salt.

According to the process of the present invention, when using a sulfoxide in admixture with another solvent it is possible to drastically reduce the chromium and aloe-emodin content to a level lower than 30 ppm.

The purification of anthrakinone derivatives of formula (I) comprises a salification/desalting step and a subsequent crystallization.

The anthrakinone derivative of formula (I), coming from the oxidizing step, is first purified through a salification/desalting step, then crystallized in a sufoxide mixture, preferably DMSO, and another solvent, preferably ketone, alcohol, acetate, ether; more preferably ketone, even more preferably acetone.

In the salification process, any alkali/alkaline-earth base, known to the skilled in the art, can be used; preferably the base is an alkali base, more preferably sodium or acetate potassium, most preferably acetate potassium. The use of other bases, such as amines, for example triethyl amine, 4-methylmorpholine, gives qualitatively worse results.

The carboxylic salt of the anthrakinone derivative of formula (I) is then converted again in the corresponding acid for treatment with a mineral acid, for example HCl, H₂SO₄, H₃PO₄, preferably HCl, in an organic solvent or in an organic solvent/water mixture. Any organic solvent known to the skilled in the art can be used, for example alcohol, such as methanol, ethanol, isopropanol; ketone, such as acetone, methyl isobutyl ketone, methyl ethyl ketone; acetate, such as ethyl acetate, butyl acetate; ether, for example tetrahydrofuran, tetrahydropyran; sulphoxide, such as DMSO; and the like.

According to a preferred process, the anthrakinone derivative of formula (I) is suspended in a sulphoxide or in a mixture of a sulphoxide, preferably DMSO, and another solvent.

Any non-toxic organic solvent well known to the skilled in the art, can be used, preferably ketone, alcohol, acetate, ether.

An alkali or alkaline-earth base is added to give the corresponding carboxylic salt.

The carboxylic salt is separated by any method known to the skilled in the art, for example by filtration; the carboxylic salt is converted into the corresponding acid by treatment with a mineral acid, in an organic solvent or in a mixture of an organic solvent and water, to obtain the compound of formula (I). This compound is then dissolved in a sulfoxide mixture, preferably DMSO, and another non-toxic solvent, preferably ketone, alcohol, acetate, ether; more preferably ketone, even more preferably acetone at a temperature ranging between 50°C and 140°C, preferably between 60°C and 120°C and then crystallized. The crystallized product is filtered by washing with DMSO or with another non-toxic organic solvent well known to the skilled in the art.

Preferably, the crude anthrakinone derivative of formula (I) is suspended in 2-20 volumes of DMSO and acetone, preferably in 2-10 volumes, and then 1-10 equivalents of an alkali /alkaline-earth base are added, preferably 1-3 equivalents.

The resulting suspension is maintained under stirring at a temperature ranging between 15°C and 100°C, preferably between 15°C and 30°C, for a time comprised between 1 and 6 hrs. preferably between 2 and 4 hrs.

The carboxylic salt of the anthrakinone derivative of formula (I) is separated by any process known to the skilled in the art, for example, by filtration. The carboxylic salt of the anthrakinone derivative of formula (I), is converted again in the corresponding acid for treatment with a mineral acid, preferably HCl, in an organic solvent or in a mixture of an organic solvent and water.

The acid compound of formula (I), is then dissolved in a mixture of DMSO and acetone and then crystallized.

The process according to the present invention can be repeated until the desired quality and purity of the product are obtained, in particular to achieve an aloe-emodin and chromium content in line with the existing regulatory provisions.

The number of crystallization steps, which depends on the solvent, on the alkali/ alkaline-earth base and on the solvent/anthrakinone derivative of formula (I) ratio, can be easily determined by the man skilled in the art, depending on the aloe-emodin and chromium content of the final product. The loss of product at each crystallization step never exceeds 20% of the total content (expressed as activity). Preferably, the process according to this invention is used to purify crude diacerein.

Crude diacerein has been purified according to the above mentioned process. Table 1 shows, as an example, the percentage of aloe-emodin after each crystallization step. The aloe-emodin content has been evaluated through the HPCL analysis.

**Table 1**

| **Test ref.** | **HPLC Analysis: % area** | | | | |
|---|---|---|---|---|---|
| | **Triacetyl aloeemodin** | **Monoacetyl rein-1^{a}** | **Monoacetyl rein-2^{b}** | **diacerein** | **rein** |
| (I) crude | 2 | n.d. | n.d. | 96,0 | n.d |
| salification/desalting | 0,05 | n.d | n.d | 99,9 | n.d |
| 1^{st} crystall. | 0,02 | n.d | n.d | 100.0 | n.d |
| 2^{nd} crystall. | 0,006 | n.d | n.d | 100.0 | n.d |
| 3^{rd} crystall. | 0,004 | n.d | n.d | 100.0 | n.d |
| 5^{th} crystall. | 0.0009 | n.d | n.d | 100.0 | n.d |

| | | | | | |
|---|---|---|---|---|---|
| n.d.: not determinable ^{a, b}: derivatives of rein mono acetylation | | | | | |

According to another object, the invention relates to the use of a sulfoxide and another solvent as a crystallization solvent for purifying anthrakinone derivatives of formula (I), preferably diacerein.

The process according to the present invention allows to obtain anthrakinone derivatives of formula (I), preferably diacerein, with high yields and above all with a degree of purity in line with the existing regulatory provisions.

The process according to the present invention allows to obtain anthrakinone derivatives of formula (I), with an aloe-emodin content <5ppm, preferably < 2ppm and a chromium content <5ppm, preferably <2ppm.

The anthrakinone derivatives of formula (I) are stable under the purification conditions of the present invention: no impurity resulting from partial hydrolysis of the acyl groups has been detected. The compounds obtained according to the process of the present invention can be used directly for the preparation of pharmaceutical compositions.

The purification process according to the present invention is economic, easy to carry out, with high yields, useful for the preparation on a large scale. The cheapness, safety, non-flammability, the low toxicity for humans and environment, the low environmental impact of the solvent used make this process very convenient and suitable for industrial exploitation.

Although the present invention has been described in its characteristic features, modifications and equivalents that are apparent to the man skilled in the art, are included in the present invention.

Below, the present invention will be illustrated by means of some examples, which should not be considered as limiting the scope of the invention.

### EXAMPLES

The diacerein purity has been determined by HPLC chromatographic column Symmetry Shield 5µ RP-8, 250 x 4.6 mm (thermostated at 30°C), isocratic mobile phase Acetonitrile 50 ml., Methanol 550 ml., acetic acid 1% 400 ml., flow: 1 ml/min., UV detector at 256 nm, 10 µl. injection.

Diacerein is eluted at about 12 minutes; relative retention time (rrt)=1;

rrt triacetyl aloe-emodin: about 0.8;

rrt monoacetil rein-1 : about 1.5;

rrt monoacetil rein-2: about 2.0;

rrt rein: about 2.4;

Low contents of aloe-emodin (few ppm) have been determined by a specific method: chromatographic column Symmetry Shield 5µ RP-18, 250 x 4.6 mm (thermostated at 30°C), isocratic mobile phase 600ml methanol; ammonium acetate 0,1M 400 ml.; flow: 1,5 ml./min.; UV detector at 256 nm; 20 µl. injection.

The chromium content has been determined by ICP (inductively coupled plasma)

The following abbreviations refer respectively to the definitions below:
AcOK (potassium acetate); DMSO (dimethylsulfoxide); hrs (hours), LOD (loss on drying)

### Example 1

To the crude diacerein (64 g., containing 3,2% of tri-acetyl aloe-emodin) in DMSO (600 ml.), AcOK anhydro (36 g.) is added.

The suspension is stirred at room temperature for four hours. Then it is filtered and the filtrate is washed with DMSO (50ml.). The wet product (100 g.; LOD 36,2%) is suspended again in DMSO (200 ml.). 37% HCl is added drop wise until a pH value of 1 is achieved (18 g. about), while maintaining temperature under 30°C. The suspension is stirred for 1 hour at room temperature, then water (90 ml.) is added drop wise and the suspension is further stirred for one hour at room temperature.

The suspension is filtered and the filtrate is washed successively with DMSO and then with water. After drying under vacuum at 90° C, 102 g. diacerein containing 0,12% of tri-acetyl aloe-emodin are obtained.

### Example 2

Diacerein (95 g.), purified according to example 1, is suspended in a 1:1 mixture of acetone-DMSO (380 ml.). The mixture is heated to reflux (75° C) to obtain a slightly opalescent solution which is hot filtered. The solution is then cooled to 0-5°C and filtered, thus isolating diacerein.

The product is not dried but used as such in the subsequent steps. The wet product (equal to 83,3 g. dry) is suspended again in the 1:1 mixture of acetone-DMSO (4 volumes), and the operation is repeated thus isolating the diacerein (equal to 67 g. dry). The whole process is repeated as many times as necessary to obtain the desired tri-acetyl aloe-emodin content (<30 ppm).

### Example 3

The purified diacerein obtained according to example 1, using DMSO containing 20% by volume of acetone, (164 kg. wet with a loss on drying of about 30% and a tri-acetyl aloe-emodin content < 0.05%), is purified by repeated crystallizations, by dissolution at 60-70°C and subsequent cooling up to room temperature, to have the product recrystallized.

During the crystallization steps about 360 kilos of DMSO and 80 It. of acetone are used. The product is collected and washed with acetone. After drying, about 50 kilos of diacerein are obtained with a purity equal to 100% and a residual tri-acetyl-aloe-emodin content lower than 2ppm.

## Claims

1. A process for obtaining anthraquinones derivatives of formula (I) or salts thereof 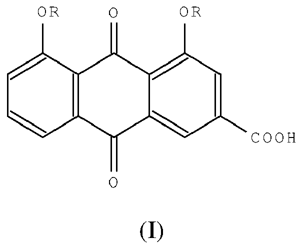 wherein R is hydrogen, acyl,
with a content of aloe-emodine, chromium < 30 ppm comprising
a) suspending the anthraquinone derivative of formula (I) in a sulfoxide or in a mixture of a sulfoxide and another solvent;
b) adding an alkali or alkaline earth base to give the corresponding carboxylic salt;
c) separating the carboxylic salt;
d) adding a mineral acid in an organic solvent or in a mixture of an organic solvent and water to give the compound of formula (I);
e) crystallisating in a mixture of a sulfoxide and another solvent the compound of formula (I) as obtained in step d).

2. Process according to claim 1 wherein R is acetyl.

3. Process according to claim 1 wherein the content of aloe-emodine, chromium is < 5 ppm, preferably < 2 ppm.

4. Process according to claim 1, step b), wherein the base is an alkali base, preferably sodium or potassium acetate, more preferably potassium acetate.

5. Process according to claim 1, step d), wherein the mineral acid is HCl, H₂SO₄, H₃PO₄, preferably HCl, and the organic solvent is selected from an alcohol, ketone, acetate, ether, sulfoxide, preferably sulfoxide, more preferably DMSO.

6. Process according to claim 1, wherein the sulfoxide is DMSO and the solvent is selected from a ketone, alcohol, acetate, ether, and the like; preferably a ketone, more preferably acetone.

7. Process according to claim 1 step e), wherein the ratio derivatives of formula (I)/mixture of a sulfoxide and another solvent ranges from 2 and 20 solvent volume, preferably 2 and 10 solvent volume.

8. Process according to claim 1 step e), wherein the derivative of formula (I) is dissolved in a mixture of a sulfoxide and another solvent at a temperature between 50°C and 140°C, preferably between 60°C and 120°C.

9. Use of a mixture of a sulfoxide and another solvent as crystallization solvent to purify raw anthraquinones derivatives of formula (I), as defined in claim 1.

10. Use according to claim 9 werein the sulfoxide is DMSO and the solvent is selected from a ketone, alcohol, acetate, ether, and the like; preferably a ketone, more preferably acetone.
